(19) Europäisches Patentamt
European Patent Office
Office européen des brevets

(11) Veröffentlichungsnummer: **0 041 661**
**B1**

# (12) EUROPÄISCHE PATENTSCHRIFT

(45) Veröffentlichungstag der Patentschrift: **18.07.84**

(51) Int. Cl.³: **C 07 C 69/757** // C07C67/32, C07F7/18, C07D307/935

(21) Anmeldenummer: **81104119.3**

(22) Anmeldetag: **29.05.81**

(54) **Neues Verfahren zur Herstellung von Carbacyclin-Zwischenprodukten.**

(30) Priorität: **06.06.80 DE 3021895**

(43) Veröffentlichungstag der Anmeldung: **16.12.81 Patentblatt 81/50**

(45) Bekanntmachung des Hinweises auf die Patenterteilung: **18.07.84 Patentblatt 84/29**

(84) Benannte Vertragsstaaten: **AT BE CH DE FR GB IT LI LU NL SE**

(56) Entgegenhaltungen:
**EP - A - 0 011 591**
**FR - A - 2 419 271**
**FR - A - 2 422 635**

**CHEMICAL ABSTRACTS, Band 91, Nr. 21, 19. November 1979, Seite 624, Nr. 174910r Columbus, Ohio, U.S.A.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Skuballa, Werner, Dr., Olwenstrasse 25, D-1000 Berlin 28 (DE)**
Erfinder: **Radüchel, Bernd, Dr., Gollanczstrasse 132, D-1000 Berlin 28 (DE)**
Erfinder: **Vorbrüggen, Helmut, Prof., Wilkestrasse 7, D-1000 Berlin 27 (DE)**

**EP 0 041 661 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zur Herstellung von Carbacyclin-Zwischenprodukten aus 7-Hydroxy-2-oxa-bicyclo[3.3.0]octan-3-ylidenessigsäureestern. Mit diesem Verfahren lassen sich in vorteilhafter Weise stabile und pharmakologisch wirksame Carbacyclin-Derivate herstellen.

In der DE-OS 2 912 409 wird eine Carbacyclin-Synthese beschrieben, die von einem bicyclischen Lakton ausgehend über Öffnung des Laktonringes und Einführung eines Essigsäureesters unter Bildung eines Dicarbonsäureesters nach vorangegangener Hydrierung schließlich unter Cyclisierung zum Carbacyclin-Zwischenprodukt führt. Diese Synthese ist nicht nur mit erheblichem synthetischen Aufwand verbunden, sondern sie verläuft bei einigen Stufen auch unter deutlichen Ausbeuteverlusten, z. B. bei der Einführung des Essigsäuremethylesterrestes in das geöffnete Ringlakton, besonders jedoch bei der Cyclisierung des Dicarbonsäurediesters und der sich anschließenden Decarboxylierung.

Es wurde nun gefunden, daß die Prostacyclin-Vorstufe 7-Hydroxy-2-oxabicyclo-[3.3.0]octan-3-yliden-essigsäureester nach vorangegangener Oxidation mit Basen (und nachfolgender Reduktion) zu dem Carbacyclin-Zwischenprodukt 7-Hydroxy-3-oxo-bicyclo[3.3.0]octan-2-yl-carbonsäureester in hoher Ausbeute umgesetzt werden kann.

Die Erfindung betrifft somit ein Verfahren zur Herstellung von 3-Oxo-7-hydroxy-bicyclo[3.3.0]-octan-2-yl-carbonsäureestern der allgemeinen Formel I

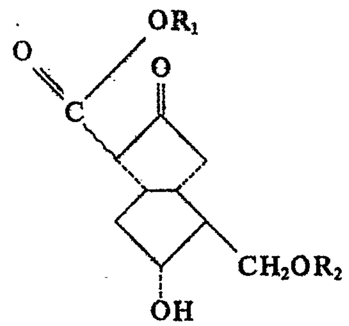

(I)

worin

$R_1$ einen gesättigten Alkylrest mit 1—6 C-Atomen oder einen Aralkylrest mit 7—10 C-Atomen und
$R_2$ ein Wasserstoffatom, einen gesättigten Alkylrest mit 1—6 C-Atomen, einen Aralkylrest mit 7—10 C-Atomen, einen Tetrahydropyranyl-, Tetrahydrofuranyl- oder einen α-Äthoxyäthylrest, einen Trialkylsilylrest mit 1—4 C-Atomen im Alkyl, wobei Alkyl auch mit Phenyl substituiert sein kann, oder den Rest

mit $R_3$ in der Bedeutung einer Alkylgruppe mit 1—6 C-Atomen oder einer Arylgruppe darstellt,

dadurch gekennzeichnet, daß man einen 7-Hydroxy-2-oxa-bicyclo[3.3.0]octan-3-yliden-essigsäureester der allgemeinen Formel II

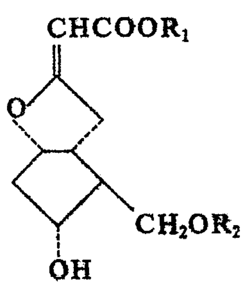

(II)

in der $R_1$ und $R_2$ die obigen Bedeutungen besitzen, nach vorangegangener Oxidation mit Collins-Reagenz, Jones-Reagenz, Pyridinium-chlorochromat oder Pyridinium-dichromat oder nach der Moffat-Pfitzner-Methode nacheinander mit 1,5-Diazabicyclo[4.3.0]non-5-en (DBN), 1,8-Diazabicyclo[5.4.0]-undec-7-en (DBU), Kalium-tert.-butylat oder Natrium-tert.-butylat und Natriumborhydrid, Lithium-tri-tert.-butoxy-aluminiumhydrid, Zinkborhydrid oder Aluminiumisopropylat umsetzt.

Verbindungen der allgemeinen Formel I sind hervorragend geeignete Zwischenprodukte zur Herstellung von stabilen, pharmakologisch wirksamen Carbacyclinderivaten, in denen der 9-Äthersauerstoff der Prostacycline durch eine Methylengruppe ersetzt ist.

Die Oxidation der Hydroxygruppe in der Verbindung der allgemeinen Formel II wird nach den dem Fachmann bekannten Methoden vorgenommen. Als Oxydationsmittel oder -methoden können beispielsweise dienen: Collins-Reagenz (Tetrahedron Letters, 1968, 3368), Jones-Reagenz (J. Chem. Soc. 1953, 2555), Pyridinium-chlorochromat (Tetrahedron Letters, 1975, 2647, Pyridinium-dichromat (Tetrahedron Letters, 1979, 399) oder die Moffat-Pfitzner-Methode. Die Oxidation wird mit Collins-Reagenz bei —20°C bis +30°C, vorzugsweise 0—5°C oder mit Jones-Reagenz bei —40°C bis +30°C, vorzugsweise —30°C bis 0°C oder mit Pyridiniumchlorochromat oder Pyridinium-dichromat bei —20°C bis +40°C, vorzugsweise bei 20°C—30°C in einem gegenüber Oxydationsmittel inerten Lösungsmittel durchgeführt. Als Lösungsmittel können je nach dem verwendeten Reagenz Methylenchlorid, Chloroform, Äthylenchlorid, Dimethylformamid, Pyridin u. a. verwendet werden. Das in praktisch quantitativer Ausbeute erhaltene Keton, in dem $R_1$ und $R_2$ die obenangegebenen Bedeutungen haben, wird ohne weitere Reinigung umgesetzt.

Als Basen für die weitere Umsetzung kommen beispielsweise in Frage: tertiäre Amine, wie 1,5-Diazabicyclo[4.3.0]non-5-en (DBN) und 1,8-Diazabicyclo[5.4.0]undec-7-en (DBU), Kalium-tert.-butylat, Natrium-tert.-butylat. Besonders bevorzugte Basen sind DBN und DBU. Die Umsetzung kann mit oder ohne Lösungsmittelzusatz bei —40°C bis +60°C, vorzugsweise 0—30°C, erfolgen. Als Lösungsmittel kommen in Frage: Tetrahydrofuran, Diäthyläther, Dioxan, Toluol. Die anschließende Reduktion der Ketogruppe wird im gleichen Reaktionsgefäß durch Zusatz von üblichen Reduktionsmitteln bei Temperaturen zwischen —80°C und +30°C, vorzugsweise bei —30°C bis +10°C, durchgeführt. Als Reduktionsmittel eignen sich beispielsweise Natriumborhydrid, Lithium-tri-tert.-butoxy-aluminiumhydrid, Zinkborhydrid, Aluminiumisopropylat usw., vorzugsweise Natriumborhydrid. Zur Verbesserung der Löslichkeit kann zweckmäßigerweise ein Alkohol, wie beispielsweise Methanol, Äthanol, Propanol, Isopropanol, zugesetzt werden.

Obwohl die dünnschichtchromatographische Untersuchung des Rohproduktes der Verbindung der allgemeinen Formel I nur geringfügige Begleitstoffe zeigt und eine Reinigung nicht notwendig erscheinen läßt, kann gegebenenfalls chromatographisch gereinigt werden. Dabei werden die reinen Verbindungen der allgemeinen Formel I in einer Gesamtausbeute von ca. 70% d. Th. aus den Verbindungen der allgemeinen Formel II erhalten.

Unter gesättigten Alkylresten mit 1—6 C-Atomen versteht man geradkettige oder verzweigte Alkylreste, wie z. B. Methyl, Äthyl, n-Propyl, Isopropyl, n-Butyl, Isobutyl, sek.-Butyl-, tert.-Butyl, n-Pentyl, Isopentyl, tert. -Pentyl-Neopentyl, n-Hexyl, Isohexyl usw. Besonders bevorzugt sind Alkylreste mit 1—4 C-Atomen.

Als Aralkylrest mit 7—10 C-Atomen kommen in Betracht: Phenylalkylreste, die im Alkyl auch verzweigt sein können, wie z. B. Phenylmethyl, Phenäthyl, $\alpha$-Phenyläthyl, Phenylpropyl, Phenylbutyl, $\alpha$-Phenylpropyl, $\alpha$-Phenylbutyl. Phenylalkylreste mit 7—8 C-Atomen kommen bevorzugt in Betracht.

Die Alkylreste der Trialkylsilyl-Gruppe in $R_2$ stellen gesättigte geradkettige oder verzweigte Alkylreste dar, die auch durch Phenyl substituiert sein können (z. B. 2-Phenyl-butyl, 3-Phenylpropyl, Phenylmethyl), wie z. B. Methyl, Äthyl, n-Propyl, Isopropyl, n-Butyl, Isobutyl, sek.-Butyl, tert.-Butyl.

Als Arylgruppen in der Bedeutung von $R_3$ sind gemeint: Phenyl, $\alpha$- und $\beta$-Naphthyl, Biphenylyl sowie mit Chlor, Brom, $C_{1-4}$-Alkyl oder $C_{1-4}$-Alkoxy substituiertes Phenyl. Als Alkylreste mit 1—4 C-Atomen kommen in Frage: Methyl, Äthyl, n-Propyl, Isopropyl, n-Butyl, Isobutyl, sek.-Butyl, tert.-Butyl. Als Alkoxyreste mit 1—4 C-Atomen kommen in Frage: Methoxy, Äthoxy, n-Propoxy, Isopropoxy, n-Butoxy, Isobutoxy, tert.-Butoxy.

Das für das erfindungsgemäße Verfahren benötigte Ausgangsmaterial der allgemeinen Formel II läßt sich aus dem bekannten Lakton der allgemeinen Formel III herstellen, worin $R_2$ die obige Bedeutung aufweist

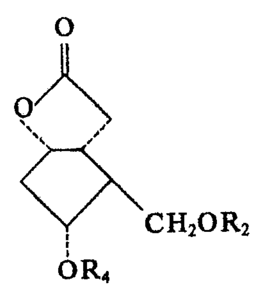

(III)

und $R_4$ ein Wasserstoffatom oder den Rest

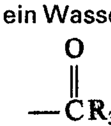

mit $R_3$ in der Bedeutung einer Alkylgruppe mit 1—6 C-Atomen oder einer Arylgruppe darstellt, in dem man mit einem Lithiumessigsäureester der allgemeinen Formel IV, worin $R_1$ die obige Bedeutung besitzt, bei Temperaturen zwischen —80° C und 0° C, vorzugsweise bei —70° C bis —50° C,

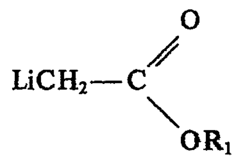

(IV)

in einem für metallorganische Reaktionen geeigneten Lösungsmittel umsetzt. Als Lösungsmittel kommen beispielsweise in Frage: Diäthyläther, Tetrahydrofuran, Dioxan, Dimethoxyäthan usw.

Zur Wasserabspaltung behandelt man das Rohprodukt der metallorganischen Umsetzung mit einer katalytischen Menge einer Säure in einem mit Wasser nicht mischbaren Lösungsmittel, wie beispielsweise Toluol, Methylenchlorid, Chloroform, Tetrachlorkohlenstoff, Diäthyläther usw., vorzugsweise Toluol, bei Temperaturen zwischen 20° C und 100° C, vorzugsweise 20° C bis 30° C. Als Säuren kommen beispielsweise in Frage: p-Toluolsulfonsäure, Schwefelsäure, Salzsäure, Bortrifluorid usw.

Die Verseifung der gegebenenfalls vorhandenen Acylgruppen erfolgt beispielsweise mit Alkali- oder Erdalkali-carbonaten in einem Alkohol. Als Alkohole kommen aliphatische Alkohole in Betracht, wie zum Beispiel Methanol, Äthanol, Butanol usw., vorzugsweise Methanol. Als Alkali- oder Erdalkalicarbonate sind beispielsweise geeignet: Kaliumcarbonat, Natriumcarbonat, Calciumcarbonat, Bariumcarbonat usw. Die Umsetzung erfolgt bei —10° C bis 70° C, vorzugsweise bei 25° C.

Die weitere Umwandlung der Verbindungen der allgemeinen Formel I in Carbacyclin-Derivate erfolgt nach bekannter Verfahrensweise, beispielsweise durch Decarbalkoxylierung mit beispielsweise 1,4-Diazabicyclo[2.2.2]octan in Toluol, Veresterung mit beispielsweise Benzoylchlorid oder Essigsäureanhydrid und Abspaltung der Schutzgruppe.

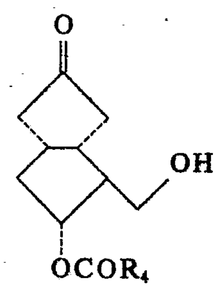

(V)

Das dabei erhaltene Keton der allgemeinen Formel V, worin $R_4$ die obenangegebene Bedeutung besitzt, läßt sich beispielsweise nach der Arbeitsweise in DE-OS 2 912 409 oder DE-OS 2 845 770 in die gewünschten Carbacycline überführen.

## Beispiel 1

### [(1S,2RS,5S,6S,7R)-7-Hydroxy-3-oxo-6-(dimethyl-tert.-butyl-silyloxymethyl)-bicyclo[3.3.0]-octan-2-yl]-carbonsäureäthylester

Zu einer Lösung von 540 g Collins-Reagenz in 4,6 ltr. Methylenchlorid (abs.) fügt man bei Eisbadtemperatur eine Lösung aus 94,4 g (1S,5R,6S,7R)-7-Hydroxy-6-(dimethyl-tert.-butyl-silyloxymethyl)-2-oxa-bicyclo[3.3.0]octan-3-yliden-essigsäureäthylester in 1,6 ltr. Methylenchlorid und rührt 15 Minuten bei 5° C. Anschließend verdünnt man mit 10 ltr. Äther, rührt die organische Phase viermal mit 5%iger Natriumbicarbonatlösung, einmal mit Wasser, zweimal mit 10%iger Schwefelsäure und dreimal mit Wasser. Man trocknet über Natriumsulfat und dampft im Vakuum ein.

Das auf diese Weise erhaltene Keton (95 g) löst man in 320 ml Tetrahydrofuran, versetzt bei 0° C mit 134 ml 1,5-Diazabicyclo[4.3.0]non-5-en, rührt 105 Minuten bei 0° C, kühlt auf —20° C, fügt 10,2 g Natriumborhydrid und anschließend 385 ml Methanol zu, rührt 100 Minuten bei —20° C, versetzt mit 160 ml Aceton, rührt 30 Minuten und gießt dann auf 2 ltr. 10%ige Zitronensäurelösung. Man extrahiert mit Äther, wäscht die organische Phase mit Wasser neutral, trocknet über Natriumsulfat und dampft im Vakuum ein. Nach Chromatographie des Rückstandes an Kieselgel erhält man mit Hexan/Äther (3 + 2)

65,5 g der Titelverbindung als farbloses Öl.

IR ($CHCl_3$): 3520, 2955, 2930, 2860, 1750, 1720, 1660, 1620, 834 $cm^{-1}$.

Das Ausgangsmaterial für die obige Titelverbindung wurde wie folgt hergestellt:

1a) (1S,5R,6S,7R)-7-Benzoyloxy-6-(dimethyl-tert.-butyl-silyloxymethyl)-2-oxabicyclo[3.3.0]octan-3-on

Zu einer Lösung von 100 g (1S,5R,6S,7R)-7-Benzoyloxy-6-hydroxy-methyl-2-oxabicyclo[3.3.0]octan-3-on und 62 g Imidazol in 110 ml Dimethylformamid fügt man bei 0°C 65 g tert.-Butyl-dimethylsilylchlorid und rührt 24 Stunden bei 24°C unter Argon. Anschließend verdünnt man mit 6 ltr. Äther, schüttelt zweimal mit 5%iger Schwefelsäure, viermal mit Wasser, trocknet über Magnesiumsulfat und dampft im Vakuum ein. Den Rückstand kristallisiert man aus Hexan/Methylenchlorid um. Dabei erhält man 137 g des Silyläthers als farblose Kristalle (F. 75°C).

1b) (1S,5R,6S,7R) -7-Hydroxy-6-(dimethyl-tert.-butyl-silyloxy-methyl)-2-oxabicyclo[3.3.0]octan-3-yliden-essigsäureäthylester

Man tropft zu 95 ml Diisopropylamin bei −25°C unter Argon 425 ml einer 1,6molaren Butyllithiumlösung in Hexan und rührt 1 Stunde bei −25°C. In diese Mischung tropft man bei −70°C 66 ml Essigsäureäthylester, rührt 20 Minuten und fügt anschließend eine Lösung von 66 g des nach Beispiel 1a hergestellten Silyläthers in 500 ml Diäthyläther und 500 ml Tetrahydrofuran zu. Man rührt 20 Minuten bei −70°C, gießt die Reaktionsmischung auf eine Mischung aus 1,4 ltr. gesättigte Ammoniumchloridlösung und 1,4 ltr. 10%ige Zitronensäurelösung, extrahiert mit Äther, wäscht den organischen Extrakt im Wasser neutral, trocknet über Magnesiumsulfat und dampft im Vakuum ein.

Den Rückstand löst man in 1,5 ltr. Toluol, fügt 1,5 g p-Toluolsulfonsäure zu, rührt 2 Stunden bei 25°C und dampft im Vakuum bei 40°C ein. Man fügt 24 g Kaliumcarbonat (wasserfrei) zu, versetzt mit 500 ml Methanol und rührt 3 Stunden bei 25°C unter Argon. Anschließend verdünnt man mit Äther, wäscht mit Sole neutral, trocknet mit Natriumsulfat und dampft im Vakuum ein. Nach Chromatographie des Rückstandes an Kieselgel erhält man mit Hexan/Essigester (7 + 3) 48 g der Titelverbindung als farblose Kristalle (F. 35°C).

IR ($CHCl_3$): 3520, 2955, 2930, 2860, 1742, 1635, 832 $cm^{-1}$.

## Beispiel 2

[(1S,2RS,5S,6S,7R)-6-Benzyloxymethyl-7-hydroxy-3-oxo-bicyclo[3.3.0]octan-2-yl]-carbonsäureäthylester

Zu einer Lösung von 30 g Collins-Reagenz in 250 ml Methylenchlorid fügt man bei Eisbadtemperatur eine Lösung von 5 g (1S,5R,6S,7R)-6-Benzyloxymethyl-7-hydroxy-2-oxabicyclo[3.3.0]octan-3-yliden-essigsäureäthylester (Beispiel 2a) in 90 ml Methylenchlorid und rührt 15 Minuten bei 5°C. Anschließend verdünnt man mit 600 ml Äther, schüttelt viermal mit 5%iger Natriumbicarbonatlösung, einmal mit Wasser, zweimal mit 10%iger Schwefelsäure und dreimal mit Wasser. Man trocknet über Magnesiumsulfat und dampft im Vakuum ein.

Den Rückstand (5 g) löst man in 18 ml Tetrahydrofuran, versetzt bei 0°C mit 7,5 ml 1,5-Diazabicyclo[4.3.0]non-5-en, rührt 100 Minuten bei 0°C, kühlt auf −20°C, fügt 550 mg Natriumborhydrid und 18 ml Methanol zu, rührt 100 Minuten bei −20°C, versetzt mit 10 ml Aceton, rührt 30 Minuten und gießt dann auf 100 ml 10%ige Zitronensäurelösung. Man extrahiert mit Äther, wäscht die organische Phase mit Wasser neutral, trocknet über Magnesiumsulfat und dampft im Vakuum ein. Nach Chromatographie des Rückstandes an Kieselgel erhält man mit Äther/Hexan (3+2) 3,4 g der Titelverbindung als farbloses Öl.

IR ($CHCl_3$): 3540, 2930, 2860, 1755, 1725, 1660, 1620 cm.

Das Ausgangsmaterial für die obige Titelverbindung wurde wie folgt hergestellt:

2a) (1S,5R,6S,7R)-6-Benzyloxymethyl-7-hydroxy-2-oxa-bicyclo[3.3.0]octan-3-yliden-essigsäureäthylester

Man tropft zu 69 ml Diisopropylamin bei −25°C unter Argon 550 ml einer 0,91molaren Butyllithiumlösung in Hexan und rührt 1 Stunde bei −25°C. In diese Mischung tropft man bei −70°C 49 ml Essigsäureäthylester, rührt 20 Minuten bei −70°C und fügt anschließend eine Lösung von 26,2 g (1S,5R,6S,7R)-6-Benzyloxymethyl-7-hydroxy-2-oxabicyclo[3.3.0]octan-3-on in 300 ml Diäthyläther und 300 ml Tetrahydrofuran zu. Nach 20 Minuten gießt man die Reaktionsmischung auf 1 ltr. gesättigte

Ammoniumchloridlösung, extrahiert mit Äther, wäscht den organischen Extrakt mit Sole neutral und trocknet über Magnesiumsulfat.

Den Eindampfrückstand löst man in 700 ml Toluol, fügt 0,25 g p-Toluolsulfonsäure zu, rührt 2 Stunden bei 25° C, destilliert im Vakuum bei 40° C ca. 400 ml Toluol ab, wäscht mit Wasser neutral, trocknet über Magnesiumsulfat und dampft im Vakuum ein.

Nach Chromatographie des Rückstandes an Kieselgel erhält man mit Hexan/Essigester 20,3 g der Titelverbindung als farblose Kristalle (F. 59° C aus Pentan/Äther).

IR ($CHCl_3$): 3540, 2990, 2945, 2870, 1695, 1639, 1120/cm.

Beispiel 3

(1R,5S,6S,7R)-7-Benzoyloxy-6-hydroxymethyl-bicyclo[3.3.0]octan-3-on

Zu einer Lösung von 47 g des nach Beispiel 1 hergestellten $\beta$-Ketoesters in 1,2 ltr. Toluol fügt man 157 g 1,4 Diazabicyclo(2.2.0)-octan und kocht 5 Stunden am Rückfluß unter Argon. Anschließend engt man im Vakuum ein, nimmt den Rückstand in 320 ml Tetrahydrofuran auf, versetzt bei 0° C mit 23 ml Benzoylchlorid und rührt 15 Minuten bei 0° C. Man fügt 9 ml Wasser zu, rührt 2 Stunden bei 25° C, löst den Kristallbrei in 2,5 ltr. Äther, schüttelt mehrfach mit 10%iger Schwefelsäure, zweimal mit Wasser und dampft im Vakuum ein. Den Rückstand rührt man 5 Stunden bei 30° C in einer Mischung aus Eisessig, Wasser, THF (65 + 35 + 10) und dampft anschließend im Vakuum ein. Nach Chromatographie des Rückstandes an Kieselgel erhält man mit Äther 24,2 g der Titelverbindung als farbloses Öl.

IR ($CHCl_3$): 3510, 2945, 1739, 1712, 1602, 1588, 1278/cm.

**Patentanspruch**

Verfahren zur Herstellung von 3-Oxo-7-hydroxy-bicyclo[3.3.0]-octan-2-yl-carbonsäureestern der allgemeinen Formel I

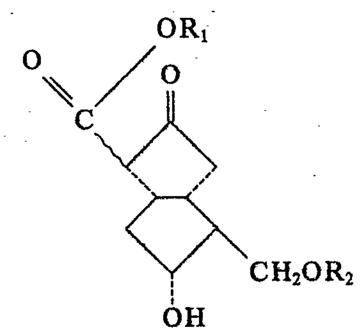

(I)

worin

$R_1$ einen gesättigten Alkylrest mit 1—6 C-Atomen oder einen Aralkylrest mit 7—10 C-Atomen und
$R_2$ ein Wasserstoffatom, einen gesättigten Alkylrest mit 1—6 C-Atomen, einen Aralkylrest mit 7—10 C-Atomen, einen Tetrahydropyranyl-, Tetrahydrofuranyl- oder einen $\alpha$-Äthoxyäthylrest, einen Trialkylsilylrest mit 1—4 C-Atomen im Alkyl, wobei Alkyl auch mit Phenyl substituiert sein kann, oder den Rest

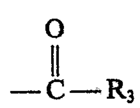

mit $R_3$ in der Bedeutung einer Alkylgruppe mit 1—6 C-Atomen oder einer Arylgruppe darstellt,

dadurch gekennzeichnet, daß man einen 7-Hydroxy-2-oxa-bicyclo[3.3.0]octan-3-yliden-essigsäureester der allgemeinen Formel II

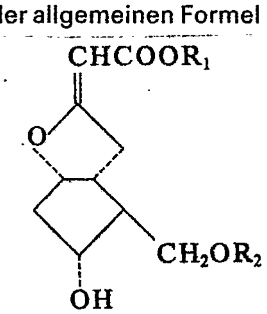

(II)

in der $R_1$ und $R_2$ die obige Bedeutungen besitzen, nach vorangegangener Oxidation mit Collins-Reagenz, Jones-Reagenz, Pyridinium-chlorochromat oder Pyridinium-dichromat oder nach der Moffat-Pfitzner-Methode nacheinander mit 1,5-Diazabicyclo[4.3.0]non-5-en (DBN), 1,8-Diazabicyclo[5.4.0]-undec-7-en (DBU), Kalium-tert.-butylat oder Natrium-tert.-butylat und Natriumborhydrid, Lithium-tri-tert.-butoxyaluminiumhydrid, Zinkborhydrid oder Aluminiumisopropylat umsetzt.

**Claim**

Process for the manufacture of 3-oxo-7-hydroxy-bicyclo[3.3.0]octan-2-ylcarboxylic acid esters of the general formula I

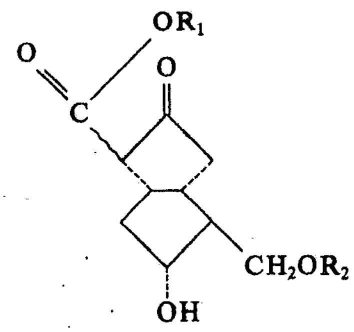

(I)

in which

$R_1$ represents a saturated alkyl radical having from 1 to 6 carbon atoms or an aralkyl radical having from 7 to 10 carbon atoms and

$R_2$ represents a hydrogen atom, a saturated alkyl radical having from 1 to 6 carbon atoms, an aralkyl radical having from 7 to 10 carbon atoms, a tetrahydropyranyl, tetrahydrofuranyl or $\alpha$-ethoxyethyl radical, or a trialkylsilyl radical having from 1 to 4 carbon atoms in each alkyl moiety, it being possible for alkyl also to be substituted by phenyl, or the radical

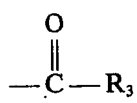

in which $R_3$ represents an alkyl group having from 1 to 6 carbon atoms or an aryl group,

characterised in that a 7-hydroxy-2-oxabicyclo[3.3.0]octan-3-ylideneacetic acid ester of the general formula II

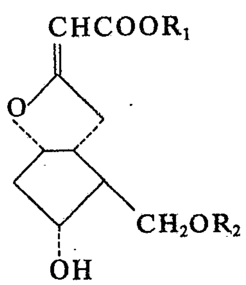

(II)

in which $R_1$ and $R_2$ have the meanings given above, after prior oxidation with Collins reagent, Jonesreagent, pyridinium chlorochromate or pyridinium dichromate or by the Moffat-Pfitzner method, are reacted in succession with 1,5-diazabicyclo[4.3.0]-non-5-ene (DBN), 1,8-diazabicyclo[5.4.0]undec-7-ene (DBU), potassium tert.-butoxide or sodium tert.-butoxide and sodium borohydride, lithium tri-tert.-butoxyaluminium hydride, zinc borohydride or aluminium isopropoxide.

**Revendication**

Procédé de préparation d'esters d'acides oxo-3hydroxy-7 bicyclo[3.3.0]octane-carboxyliques-2 répondant à la formule générale I:

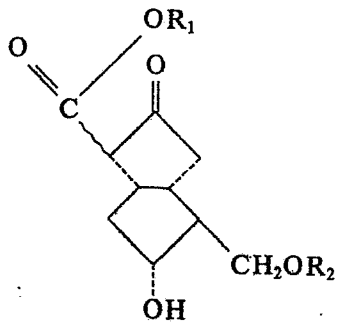

(I)

dans laquelle

$R_1$ représente un radical alkyle saturé contenant de 1 à 6 atomes de carbone ou un radical aralkyle contenant de 7 à 10 atomes de carbone et

$R_2$ représente un atome d'hydrogène, un radical alkyle saturé contenant de 1 à 6 atomes, un radical aralkyle contenant 7 à 10 atomes de carbone, un radical tétrahydropyrannyle, tétrahydrofurannyle ou $\alpha$-éthoxyéthyle, un radical trialkylsilyle dont les radicaux alkyles contiennent chacun de 1 à 4 atomes de carbone et peuvent porter chacun un radical phényle, ou représente un radical

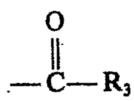

dont le symbole $R_3$ désigne un radical alkyle contenant de 1 à 6 atomes de carbone ou un radical aryle,

procédé caractérisé en ce qu'on fait réagir un ester d'acide (hydroxy-7 oxa-2 bicyclo[3.3.0]octylidène-3)-acétique répondant à la formule générale II:

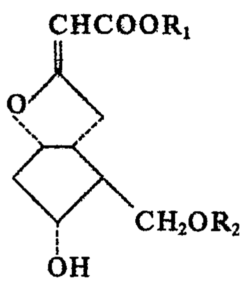

(II)

dans laquelle $R_1$ et $R_2$ ont les significations indiquées ci-dessus, après l'avoir oxydé au moyen du réactif de Collins, du réactif de Jones, du chlorochromate de pyridinium ou du dichromate de pyridinium ou par la méthode de Moffat-Pfitzner, successivement avec le diaza-1,5 bicyclo[4.3.0]nonène-5 (DBN), le diaza-1,8 bicyclo[5.4.0]undécène-7 (DBU), le tert-butylate de potassium ou le tert-butylate de sodium et avec le tétrahydruro-borate de sodium, l'hydruro-tris-tert-butoxy-aluminate de lithium, le tétrahydruro-borate de zinc ou l'isopropylate d'aluminium.